# EUROPÄISCHE PATENTSCHRIFT

(11) **EP 3 432 840 B1**
(45) Veröffentlichungstag und Bekanntmachung des Hinweises auf die Patenterteilung: **06.05.2026**
(21) Anmeldenummer: 17713296.6
(22) Anmeldetag: 24.03.2017
(51) Int. Cl.: A61F 2/78, A61F 2/80

(54) **FLEXIBLER PROTHESENLINER UND PROTHESENSCHAFTSYSTEM**
FLEXIBLE PROSTHETIC LINER AND PROSTHETIC SHAFT SYSTEM
MANCHON SOUPLE POUR PROTHÈSE ET SYSTÈME D'EMBOÎTURE

(30) Priorität: 24.03.2016 DE 102016105615
(43) Veröffentlichungstag der Anmeldung: 30.01.2019
(73) Patentinhaber: Ottobock SE & Co. KGaA, 37115 Duderstadt (DE)
(72) Erfinder: HILLMANN, Martin, 37115 Duderstadt (DE); RAUSCH, Michael, 37339 Brehme (DE); ROST, Roger, 79104 Freiburg (DE); MOSLER, Lüder, 37115 Duderstadt (DE)
(74) Vertreter: Gramm, Lins & Partner Patent- und Rechtsanwälte PartGmbB
(86) Internationale Anmeldenummer: PCT/EP2017/057067
(87) Internationale Veröffentlichungsnummer: WO 2017/162855

(56) Entgegenhaltungen:
- EP-A1- 3 355 838
- US-A- 4 923 474
- US-A1- 2010 121 464
- US-A1- 2010 198 361
- US-A1- 2015 032 226
- US-B1- 6 793 682

## Beschreibung

Die Erfindung betrifft einen flexiblen Prothesenliner mit einer proximalen Einstiegsöffnung für einen Stumpf einer Gliedmaße mit einem distalen Ende sowie einem flexiblen Zugmittel, das in einem distalen Endbereich an dem Prothesenliner angeordnet ist. Die Erfindung betrifft ebenfalls ein Prothesenschaftsystem mit einem solchen Prothesenliner und einem Prothesenschaft, der eine proximale Einstiegsöffnung, eine den Prothesenliner zumindest teilweise umfänglich umgebende Seitenwand und einen distalen Abschlussbereich aufweist.

Aus der US 6,793,682 B2 ist ein Verriegelungssystem mit einem flexiblen Liner bekannt, der einen Gliedmaßenstumpf umgibt. Dem Liner ist ein Auslass an dem unteren, distalen Ende angeordnet. Ein erster Gurt ist an einem proximalen, also oberen Ende des Prothesenliners angeordnet, an diesem ersten Gurt ist eine Schlaufe ausgebildet. Ein zweiter Gurt ist an dem Liner zentral an dessen distalen Ende verschraubt und wird aus einer Seitenwand eines Prothesenschaftes herausgeführt. An dem zweiten Gurt ist ein Klettverschluss angeordnet, sodass nach dem Durchführen des freien Endes des zweiten Gurtes durch die Schlaufe und ein Umschlagen auf sich zurück der zweite Gurt fixiert werden kann. Zum Austeigen aus dem Prothesenschaft oder zum Ablegen des Prothesenschaftes wird der zweite Gurt gelöst, aus der Schlaufe an dem ersten Gurt ausgezogen und zusammen mit dem ersten Gurt aus dem Prothesenschaft herausgeführt. Dabei wird das freie Gurtende aus der Durchführöffnung in dem distalen Schaftbereich herausgezogen.

Insbesondere für geriatrische Patienten ist das Einfädeln des freien Gurtendes durch den Prothesenschaft und die Durchgangsöffnung in dem distalen Prothesenschaftbereich mühselig.

Die US 2015/0032226 A1 betrifft ein Verbindungssystem zum lösbaren Verbinden eines Prothesenschaftes mit einem über einen Amputationsstumpf gezogenen Liner, wobei das Verbindungssystem einen Verbindungsstift und eine Aufnahmeeinrichtung mit einer Aufnahme umfasst, in die der Verbindungsstift einführbar ist. Der Verbindungsstift umfasst wenigstens zwei starre Stiftsegmente, die entlang einer Längsrichtung des Verbindungsstiftes gegeneinander verlagerbar, hintereinander angeordnet sind.

Die US 2010/0198361 A1 betrifft eine Lagerungsvorrichtung zur Verwendung mit einer Prothese mit einem äußeren Schaft und einem inneren Schaft mit einem Positionierelement, das einem Nutzer eine Ausrichtung des Innenschaftes mit dem Außenschaft ermöglicht. Ein reibungsreduzierendes Element ist ausgebildet, um das Positionierelement in Richtung auf den Nutzer zu führen, wobei das reibungsreduzierende Element ausgebildet ist, den Reibungswiderstand des Positionierelementes zu verringern. Ein Gurt kann durch den Außenschaft um das reibungsvermindernde Element herumgeführt und an der Außenseite über eine Formschlusseinrichtung festgelegt werden.

Die US 2010/0121 464 A1 betrifft ein Verankerungssystem für eine Prothese mit einem Liner zum Aufnehmen eines Amputationsstumpfes mit einem ersten Gurt, das an dem proximalen oberen Ende des Liners angeordnet ist. Ein Teil eines Klettverschlussmaterials ist an dem Gurt angeordnet. Ein zweiter Gurt ist an dem unteren Ende des Liners angeordnet und weist einen zweiten Bereich eines Klettverschlusses auf. **In** einem Prothesenschaft zur Aufnahme des Liners ist ein erster Schlitz zum Hindurchführen des ersten Gurtes und ein zweiter Schlitz zum Durchführen des zweiten Gurtes angeordnet. Die beiden Gurte können im angelegten Zustand miteinander verbunden werden.

Die US 4,923,474 A betrifft einen Prothesenliner mit einer Kopplungseinrichtung an seinem distalen Ende. Die Kopplungseinrichtung dient zur Aufnahme eines kugelförmigen Kopplungselementes, das mit einer Schnur gekoppelt ist. Die Schnur wird durch eine Öffnung in einem Prothesenschaft hindurchgezogen, bis eine Verriegelung über ein Verriegelungsmittel erfolgt.

Die nachveröffentlichte EP 3 355 838 A1 betrifft eine Festlegung eines Prothesenliners in einem Prothesenschaft über ein Zugmittel, das über eine Aufroll- oder Spanneinrichtung, die an der Außenseite des Prothesenschaftes angeordnet ist, gespannt werden kann. Das Zugmittel wird in einer Bohrung eines Stopfens gehalten und nach unten zu einem Umlenkelement geführt und seitlich zu der Spanneinrichtung geleitet. Der Stopfen als ein erstes Verbindungselement wird in ein Verbindungselement an dem Liner eingeschraubt. Alternativ kann die Befestigung der Verbindungselemente aneinander über Schnallen, Gurte, Druckknöpfe, Klettverschluss oder eine Magnetverbindung erfolgen.

Aufgabe der vorliegenden Erfindung ist es, einen verbesserten Prothesenliner sowie ein verbessertes Prothesenschaftsystem aus Prothesenliner und Prothesenschaft bereitzustellen.

Erfindungsgemäß wird diese Aufgabe durch einen flexiblen Prothesenliner mit den Merkmalen des Hauptanspruches und ein Prothesenschaftsystem mit dem Merkmal des nebengeordneten Anspruchs gelöst. Vorteilhafte Ausgestaltungen und Weiterbildungen der Erfindung sind in den Unteransprüchen, der Beschreibung sowie in den Figuren näher erläutert.

Der erfindungsgemäße flexible Prothesenliner mit einer proximalen Einstiegsöffnung für einen Stumpf einer Gliedmaße mit einem distalen Endbereich sowie einem flexiblen Zugmittel, dass in dem distalen Endbereich an dem Prothesenliner angeordnet ist, sieht vor, dass das Zugmittel über ein werkzeuglos betätigbares Verschlusssystem lösbar an dem Prothesenliner befestigt ist und dass das Verschlusssystem eine Magnetsicherung aufweist. Durch die werkzeuglose Befestigung und Trennung des Zugmittels an dem oder von dem distalen Ende des Prothesenliners ist es möglich, dass das Zugmittel, das vorzugsweise als unelastischer Gurt ausgebildet ist, an einem Prothesenschaft verbleibt, wenn der Prothesenschaft abgelegt werden soll und ein Patient aus dem Prothesenschaft aussteigt. Dadurch wird das mühselige Einfädeln des freien Endes des Zugmittels überflüssig, ein Patient muss zum Anlegen des Prothesenschaftes den Prothesenliner nur noch mit dem Zugmittel über das Verschlusssystem verbinden und das weiterhin innerhalb des Prothesenschaftes befindliche Zugmittel betätigen, um einerseits das Einführen in den Prothesenschaft zu erleichtern und andererseits eine Verriegelung gegen eine axiale Auszugsbewegung entgegen der Einstiegsrichtung zu gewährleisten. Darüber hinaus kann durch die Trennung von Liner und Zugmittel ein Austausch einer der beiden Komponenten bei Bedarf vorgenommen werden, ohne dass die noch brauchbare Komponente gewechselt werden muss.

Das Zugmittel ist insbesondere formschlüssig an dem Prothesenliner befestigt, ergänzend oder alternativ kann auch eine kraftschlüssige, insbesondere magnetische Verriegelung oder zumindest Sicherung der formschlüssigen Verriegelung durch eine kraftschlüssige, insbesondere magnetische Verriegelung erreicht werden. Magnetische Kräfte können zusätzlich zum erleichterten Einfädeln, Annähern und/oder Orientieren der Verbindungskomponenten dienen.

Das Zugmittel kann ein erstes Formschlusselement aufweisen, das mit einem zweiten Formschlusselement, das dauerhaft an dem Prothesenliner angeordnet ist, in Eingriff treten, um so eine sichere und einfach herzustellende Verbindung zwischen dem Zugmittel und dem Prothesenliner herzustellen. Die Formschlusselemente weisen zumindest einen Hinterschnitt auf und wirken dergestalt zusammen, dass das Verschlusssystem als Schnappverbindung, Klettverschluss, Druckknopf, Schiebeeinsatz, Clipsverbindung, Schlaufe und/oder Bajonettverschluss ausgebildet ist. Gegebenenfalls kann die Formschlussverbindung zwischen den beiden Formschlusselementen durch eine zusätzliche Sicherung, beispielsweise eine Drehsicherung aufgrund magnetischer Verriegelungselemente, eine Dreh-Schiebebewegung, eine Längenverringerung oder Umfangsverringerung oder eine Kombination davon gesichert werden, sodass eine ungewollte Entriegelung nicht möglich ist. Eine Entriegelungssicherung kann auch innerhalb des Prothesenschaftes angeordnet oder ausgebildet sein, beispielsweise durch Einpassen des Verschlusssystems in eine korrespondierende Ausnehmung, sodass eine Relativbewegung zwischen den in Eingriff befindlichen Formschlusselementen nach dem Einführen in einen Prothesenschaft nicht mehr möglich ist.

Das Verschlusssystem weist erfindungsgemäß eine Magnetsicherung auf. Bei einem Magnetelement kann eine Orientierung der Verschlusselemente zueinander sichergestellt werden, ebenfalls ist es möglich, durch eine Magnetkomponente eine festgelegte Relativbewegung zwischen den beiden Formschlusselementen herzuführen, sodass eine Verriegelung auch entgegen der ursprünglichen Einführrichtung stattfindet. Ein Auflösen dieser Verriegelung kann durch ein Betätigungselement oder durch Überwinden der Magnetsicherung aufgrund einer Drehbewegung, Schiebebewegung, Druckbewegung oder dergleichen erfolgen.

Das Verschlusssystem und insbesondere die darin genutzten Formschlusselemente blockieren bevorzugt eine Bewegung zwischen dem Zugmittel und dem Prothesenliner entgegen der Einführrichtung, sodass gewährleistet wird, dass der Prothesenliner an einem Prothesenschaft im distalen Bereich sicher gehalten und fixiert wird, wenn das Zugmittel an dem Prothesenschaft festgelegt wird.

Ebenso kann das Formschlusselement in zumindest einer Richtung senkrecht zu der Auszugsrichtung eine Verlagerung der Formschlusselemente zueinander blockieren, beispielsweise durch eine Schiebeeinrichtung mit Endanschlag, eine Schwalbenschwanzführung, eine Schnappverbindung oder durch andere, geeignete Hinterschnitte. Dadurch wird die räumliche Orientierung des Zugmittels an dem Prothesenliner sichergestellt, insbesondere wenn alle translatorischen Freiheitsgrade durch das Verschlusssystem blockiert sind, was grundsätzlich vorgesehen ist, beispielsweise bei kugelförmigen Schnappverbindungen oder Druckknopfverschlüssen.

An dem distalen Ende des Prothesenliners kann eine formschlüssige und/oder kraftschlüssige Verriegelungseinrichtung zur Verriegelung des Prothesenliners an einem Prothesenschaft angeordnet oder ausgebildet sein, die zusätzlich zu dem Zugmittel wirkt, um eine weitere Sicherung des Liners an einem Prothesenschaft zu bewirken.

Um zu verhindern, dass das freie Ende des Zugmittels unbeabsichtigt aus dem Prothesenschaft herausgleitet, kann an dem Ende, das dem Verschlusssystem gegenüberliegt, eine bevorzugt abnehmbare Rückhalteeinrichtung angeordnet sein, die solche Abmessungen aufweist, dass die Rückhalteeinrichtung und das Zugmittel nicht durch eine Durchtrittsöffnung in dem Prothesenschaft hindurchtreten können. Nach dem erstmaligen Durchführen des Zugmittels durch die Durchtrittsöffnung wird das Rückhalteelement an dem Zugmittel befestigt, beispielsweise in Gestalt eines aufclipsbaren oder über Klettverschluss befestigbaren Sperrelements. Das Sperrelement verhindert, dass das Zugmittel ungewollt von dem Prothesenschaft getrennt wird. An dem anderen Ende verhindert der Teil des Verschlusssystems, der an dem Zugmittel befestigt ist, ein Herausrutschen aus dem Prothesenschaft.

Das erfindungsgemäße Prothesenschaftsystem mit einem Prothesenliner, wie er oben beschrieben worden ist, und einem Prothesenschaft mit einer proximalen Einstiegsöffnung, einer einen Stumpf zumindest teilweise umfänglich umgebenden Seitenwand und mit einem distalen Abschlussbereich sieht eine Durchtrittsöffnung in dem distalen Abschlussbereich für das an dem distalen Endbereich des Prothesenliners angeordnete Zugmittel vor, sodass sowohl eine Sicherung gegen ein Herausziehen des Prothesenliners und damit des Stumpfes aus dem Prothesenschaft als auch eine Festlegung der rotatorischen Orientierung des Prothesenschaftes an dem Prothesenliner und damit an dem Prothesenstumpf gewährleistet werden kann.

Der Abschlussbereich des Prothesenschaftes kann eine Abstützeinrichtung mit einer Ausnehmung für das Verschlusssystem aufweisen, sodass das Verschlusssystem, das sich im distalen Endbereich des Prothesenliners befindet, nicht zu unerwünschten Druckerhöhungen auf den Stumpf führt. Die Abstützeinrichtung ist bevorzugt formstabil und kann schüsselartig ausgebildet sein. Die Ausnehmung kann gleichzeitig als Sicherung für die Festlegung des Zugmittels an dem Prothesenliner dienen, beispielsweise indem ein Herausgeleiten eines Formschlusselementes durch eine entsprechend positionierte Seitenwand verhindert wird.

An der Außenseite der Seitenwand des Prothesenschaftes kann eine Fixiereinrichtung für das im distalen Endbereich des Prothesenliners angeordnete Zugmittel vorgesehen sein, beispielsweise eine Schnalle, ein Klettverschluss oder ein anderes Formschlussmittel, um das Zugmittel an dem Prothesenschaft zu fixieren. Alternativ kann ein Umlenkelement an der Seitenwand fixiert werden, sodass durch ein Einführen des Zugelementes in das Umlenkelement sowie Umlenken und Fixieren auf sich selbst, beispielsweise durch einen Klettverschluss, eine Schnalle oder einen Haken, eine Fixierung des Zugmittels an dem Prothesenschaft erfolgt.

In dem Prothesenschaft kann im proximalen Bereich der Seitenwand ein Fenster als zweite Durchtrittsöffnung für einen zweiten Gurt oder für ein zweites Zugmittel vorgesehen sein, das am proximalen Endbereich des flexiblen Prothesenliners befestigt ist, sodass auch die beiden Zugmittel aneinander befestigbar sind, beispielsweise über einen Klettverschluss, Druckknöpfe, eine Schnalle oder dergleichen.

Die Durchtrittsöffnung zum Durchführen des ersten Zugmittels, das in dem distalen Endbereich des Prothesenliners über das Verschlusssystem daran festgelegt ist, ist vorzugsweise senkrecht zur Einführrichtung des Stumpfes und Prothesenliners in dem Prothesenschaft orientiert. Dadurch werden nach den Anlegen und Festlegen des Zugmittels Zugkräfte erzeugt, die in einer Horizontalebene wirken. Die Zugkräfte belasten das Verschlusssystem mit Kräften innerhalb der Horizontalebene, sodass beispielsweise durch Ausgestaltung des Verschlusssystems als Formschlusseinrichtung mit einer Schiebekomponente die Kräfte in der Horizontalebene durch einen Anschlag leicht aufgenommen werden können. Gleichzeitig kann durch den Anschlag verhindert werden, dass das Verschlusssystem ungewollt geöffnet wird. Bei einer gleichzeitigen Ausgestaltung des Verschlusssystems mit einer Formschlusskomponente, die eine Bewegung in Auszugsrichtung sperrt, wird der Prothesenliner in dem Prothesenschaft gesichert.

Bevorzugt ist die Durchtrittsöffnung lateral angeordnet, sodass das Zugmittel leicht auf der Außenseite herausgeführt, betätigt und daran festgelegt werden kann.

Nachfolgend werden Ausführungsbeispiele der Erfindung anhand der beigefügten Figuren näher erläutert. Gleiche Bezugszeichen bezeichnen gleiche Komponenten. Es zeigen:
- Figur 1-: die Komponenten eines Prothesenschaftsystems in teilmontiertem Zustand;
- Figur 2-: eine Detailansicht eines Verschlusssystems;
- Figur 2a-: eine Teilansicht eines Formschlusselementes;
- Figur 3-: eine Variante des Verschlusssystems;
- Figur 4-: eine Detaildarstellung eines Prothesenliners mit Zugmittel vor der Montage;
- Figur 5-: eine Detailansicht der Figur 4;
- Figur 6-: eine Draufsicht auf ein Verschlusssystem gemäß Figur 5;
- Figuren 7 und 8-: Varianten des montierten Prothesenschaftsystems in Schnittansicht;
- Figur 9-: eine perspektivische Darstellung eines montierten Prothesenschaftsystems; sowie
- Figuren 10a bis 10e-: Einzeldarstellungen eines Zugmittels.

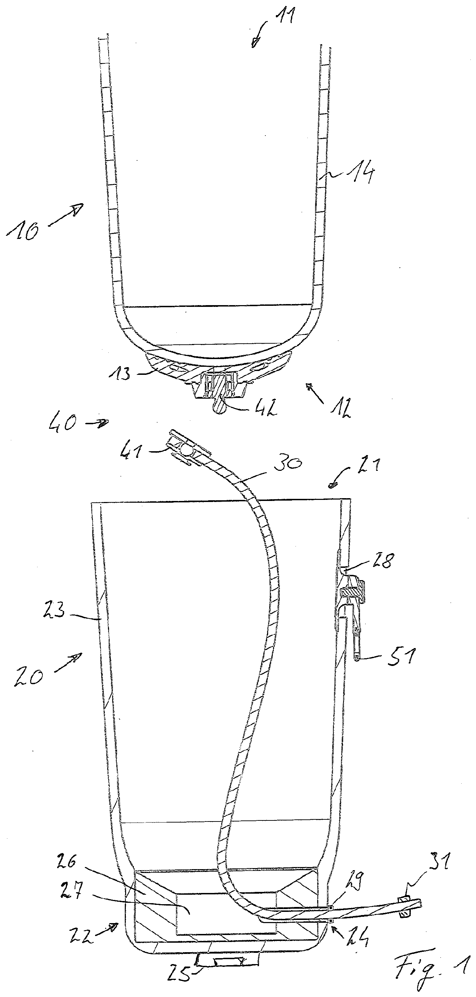

Figur 1 zeigt ein Prothesenschaftsystem mit einem flexiblen Prothesenliner 10, der eine proximale Einstiegsöffnung 11 für einen Stumpf einer Gliedmaße sowie einen distalen Endbereich 12 aufweist. Der Prothesenliner 10 ist im distalen Endbereich 12 bevorzugt geschlossen ausgebildet, auch die Seitenwand 14, die von dem distalen Endbereich 12 bis zur Einstiegsöffnung 11 reicht, kann geschlossenwandig ausgebildet sein, sodass sich der Prothesenliner 10 vollumfänglich um den nicht dargestellten Stumpf einer Gliedmaße herumlegen kann. Der Prothesenliner 10 besteht im Bereich der Seitenwand vorzugsweise aus einem Silikon oder einem anderen flexiblen oder gegebenenfalls elastischen Polymer. Grundsätzlich ist es auch möglich, dass auf der Innenseite oder auf der Außenseite des Prothesenliners 10 ein anderes Material als das Grundmaterial aufgebracht oder eingearbeitet ist, auch eine Textilbeschichtung kann auf einer der Oberflächen des Prothesenliners 10 angeordnet sein.

An dem distalen, in dem Ausführungsbeispiel geschlossenen Endbereich 12 des Prothesenliners 10 ist eine formstabile, bedingt flexible Abschlusskappe 13 angeordnet, die den distalen Abschluss des Prothesenliners 10 bildet. Die Abschlusskappe 13 dient zur Stabilisierung des distalen Endbereiches 12 und zur Ausbildung einer Auflagefläche für das Stumpfende. Innerhalb der distalen Abschlusskappe 13 ist ein zweites Formschlusselement 42 angeordnet, im dargestellten Ausführungsbeispiel eingeschraubt, das mit einem ersten Formschlusselement 41, das an einem flexiblen, bevorzugt zugstarren Zugmittel 30 in Gestalt eines Gurtes oder eines Seils befestigt ist, in Eingriff bringbar ist.

Das erste Formschlusselement 41 ist formschlüssig an dem Zugmittel 30 festgelegt, es sind jedoch auch andere Befestigungsarten möglich. Das erste Formschlusselement 41 bildet zusammen mit dem zweiten Formschlusselement 42 ein Verschlusssystem 40, wobei die beiden Formschlusselemente 41, 42 werkzeuglos voneinander trennbar und aneinander festlegbar sind, sodass das Zugmittel 30 schnell an dem Prothesenliner 10 befestigt oder von diesem getrennt werden kann.

Das Zugmittel 30 ist in einem Prothesenschaft 20 eingeführt. Der Prothesenschaft 20 weist eine proximale Einstiegsöffnung 21, einen distalen Abschlussbereich 22 und eine zumindest teilweise umlaufende Seitenwand 23 auf, über die ein Aufnahmeraum für den Prothesenliner 10 zusammen mit dem Stumpf ausgebildet wird. Der Prothesenschaft 20 kann individuell für den Nutzer angefertigt werden, beispielsweise durch Abformen des Stumpfes und Anpassen eines in Umfangsrichtung geschlossenen, der Kontur des Stumpfes folgenden einteiligen Prothesenschaft. Die Seitenwand 23 kann dabei einen geschlossenen Querschnitt aufweisen, zumindest über den größten Teil der Längserstreckung oder Höhe des Prothesenschaftes. Es können auch Fenster 28 in dem Prothesenschaft 20 beziehungsweise der Seitenwand 23 ausgebildet sein. Ebenso ist es möglich, dass der Prothesenschaft 20 aus mehreren Segmenten besteht, die an dem distalen Abschlussbereich 22 angeformt oder befestigt sind und angelegten Zustand einen formstabilen Prothesenschaft 20 ausbilden. Der Prothesenschaft 20 dient zur Aufnahme des Prothesenliners 10 mit dem Stumpf sowie zur Anordnung und Festlegung weiterer Prothesenkomponenten, beispielsweise Prothesenkniegelenke, Prothesenfüße oder Prothesenhände.

Der Prothesenschaft 20 im dargestellten Beispiel weist im distalen Endbereich 22 eine formstabile Abstützeinrichtung 26 auf, die innerhalb des Prothesenschaftes 20 angeordnet ist. Grundsätzlich ist es auch möglich, die formstabile Abstützeinrichtung 26 an der Außenseite anzuordnen. Die Abstützeinrichtung 26 weist eine schalenartige oder kappenartige Form auf und kann eine zylindrische Außenkontur aufweisen. Sie dient zur Abstützung des Liners 10 und des Stumpfes und/oder zur Stabilisierung des distalen Endes des Prothesenschaftes 20. An dem distalen Ende des Prothesenschaftes 20 ist zudem eine Einrichtung 25 zum Anschließen einer prothetischen Komponente angeordnet. Die Einrichtung 25 kann beispielsweise als Aufnahme eines Pyramidenadapters oder als Pyramidenadapter selbst ausgebildet sein.

Innerhalb der Abstützeinrichtung 26 ist eine Ausnehmung 27 ausgebildet, um das Verschlusssystem 40 im montierten und angelegten Zustand aufzunehmen, sodass an dem Stumpfende keine übermäßigen Druckbelastungen aufgrund einer zentralen, punktuellen Krafteinleitung über das Verschlusssystem 40 auftreten. Die Abschlusskappe 13 liegt großflächig auf der abgeschrägten Oberkante der Abstützeinrichtung 26 auf und bewirkt eine gleichmäßige Druckverteilung auf das Stumpfende.

In der Abstützeinrichtung 26 sowie in der Seitenwand 23 im distalen Abschlussbereich 22 des Prothesenschaftes ist eine Durchtrittsöffnung 24 für das Zugmittel 30 ausgebildet. An der Außenseite der Durchtrittsöffnung 24 ist eine Dichtung 29 angeordnet, um nach Möglichkeit eine fluiddichte Abdichtung des Innenraumes des Prothesenschaftes 20 zu gewährleisten. An dem freien Ende des Zugmittels 30 ist ein Sperrelement 31 angeordnet, das lösbar an dem Zugmittel 30 befestigt ist, um nach dem Durchführen des Zugmittels 30 durch die Durchtrittsöffnung 24 ein ungewolltes Herausziehen bei einem Aussteigen aus dem Prothesenschaft 20 zu vermeiden.

An der Außenseite der Seitenwand 23 ist ein Umlenkelement 51 angeordnet. Das Umlenkelement 51 ist als Öse ausgebildet, die an einem Montageteil befestigt ist, das in einem Fenster 28 in der Seitenwand 23 des Prothesenschaftes angeordnet ist. Das freie Ende des Zugmittels 30 wird durch das Umlenkelement 51 hindurchgezogen, wenn der angelegte Prothesenliner 10 mit dem Zugmittel 30 über das Verschlusssystem 40 gekoppelt ist. Beim Einsteigen in den Prothesenschaft 20 wird gleichzeitig das freie Ende des Zugmittels durch die Durchtrittsöffnung 24 gezogen, sodass der Prothesenliner 10 in dem Prothesenschaft 20 eingezogen wird. Nach dem Durchführen des freien Endes des Zugmittels 30 durch das Umlenkelement 51 kann über nicht dargestellte Befestigungselemente, beispielsweise Klettverschlüsse, Schlaufen oder dergleichen das Zugmittel 30 auf sich selbst fixiert werden, sodass im vollständig eingeführten Zustand das Verschlusssystem 40 mit einer Zugspannung beaufschlagt wird, die durch das gespannte Zugmittel 30 aufgebracht wird. Statt eines Umlenkelementes kann auch ein zweites Zugmittel oder ein Befestigungselement an der Seitenwand festgelegt sein, das mit der ersten Zugmittel 30 verbunden werden kann, um das erste Zugmittel 30 in der gewünschten, gespannten Stellung zu fixieren.

Im montierten Zustand befindet sich das Verschlusssystem 40 ungefähr auf der Höhe der waagerecht orientierten, zur lateralen Seite hingerichteten Durchtrittsöffnung 24 oder leicht darüber, sodass bei einer ausreichenden Spannung des Zugmittels 30 eine Fixierung des Prothesenliners 10 innerhalb des Schaftes 20 gewährleistet wird. Weder eine seitliche Bewegung in Horizontalebene noch eine Auszugsbewegung entgegen der Einführrichtung des Prothesenliners 10 ist dann möglich. Das Verschlusssystem 40, das eine formschlüssige Verriegelung durch die beiden Formschlusselemente 41, 42 bereitstellt, blockiert eine seitliche Relativbewegung der beiden Formschlusselemente 41, 42 in Horizontalebene ebenso wie eine Verlagerung in oder entgegen der Einführrichtung, sodass lediglich aufgrund der kugelkopfförmigen Ausgestaltung des zweiten Formschlusselementes 42 ein rotatorischer Freiheitsgrad zur Verfügung steht. Alle anderen Freiheitsgrade werden blockiert.

Alternativ zu einer Festlegung des Zugmittels 30 auf sich selbst und Umlenkung über ein Umlenkelement 51 kann auch eine direkte Festlegung des Zugmittels 30 an der Außenseite der Seitenwand 23 erfolgen.

Zum Aussteigen aus dem Prothesenschaft 20 wird die Fixierung des Zugmittels 30 gelöst und der Prothesenliner 10 mitsamt Stumpf aus dem Prothesenschaft 20 herausgeführt. Dabei wird das Zugmittel 30 mit aus dem Innenraum des Prothesenschaftes 20 herausgezogen. Sobald sich der Prothesenliner 10 außerhalb des Prothesenschaftes 20 befindet, kann durch Lösen der Formschlusselemente 41, 42 voneinander, beispielsweise durch Drücken einer Federverriegelung, das Zugmittel 30 von dem Prothesenliner 10 gelöst werden, sodass der Prothesenschaft 20 zusammen mit den daran befestigten übrigen Prothesenkomponenten und dem Zugmittel 30 von dem Prothesenliner 10 getrennt wird.

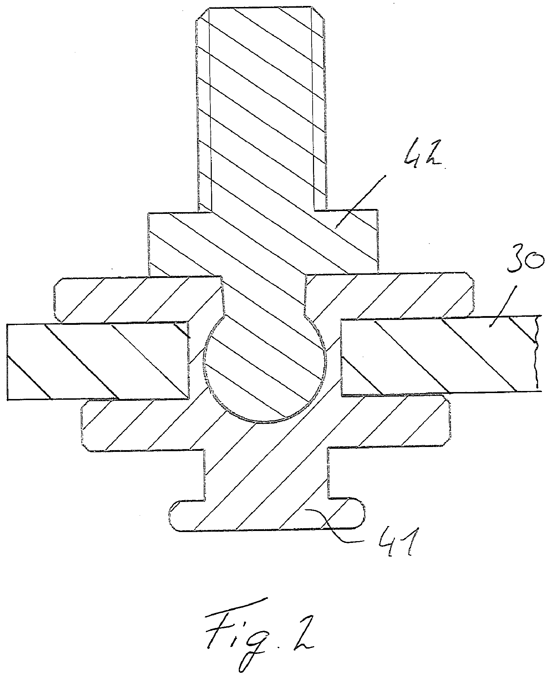

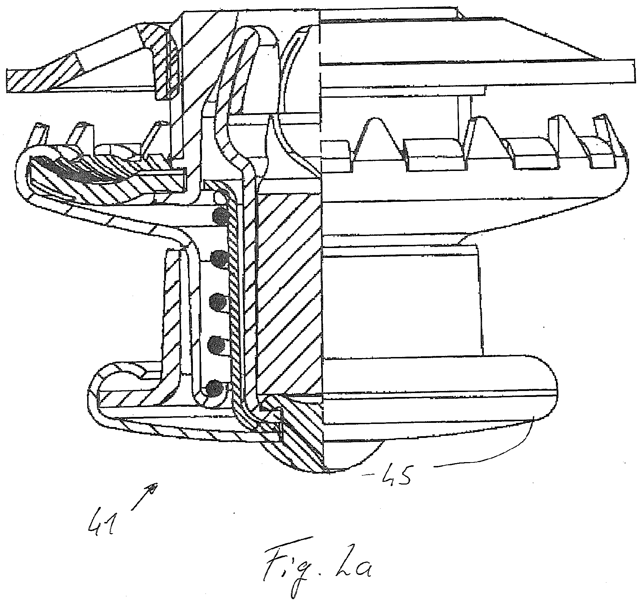

Figur 2 zeigt eine Detaildarstellung der beiden Formschlusselemente 41, 42 gemäß Figur 1 im verriegelten Zustand. Das erste Formschlusselement 41 ist an dem gurtartigen Zugmittel 30 fixiert. An dem zweiten Formschlusselement 42 ist ein Kugelkopf ausgebildet, an dessen gegenüberliegenden Ende ein Schraubgewinde angeordnet ist, sodass das zweite Formschlusselement 42 mit einem Werkzeug an dem Prothesenliner 10 befestigbar ist. Das erste Formschlusselement 41 weist eine zu dem zweiten Formschlusselement 42 korrespondierende Ausnehmung auf, in dem der Kugelkopf einschnappbar ist, um die beiden Formschlusselemente 41, 42 formschlüssig miteinander zu verriegeln. Zum Montieren wird das erste Formschlusselement 41 senkrecht auf den Kugelkopf gedrückt, das Material des ersten Formschlusselementes 41 weitet sich auf oder es sind federnde Komponenten oder Schlitze darin angeordnet, die verlagert werden, sodass nach der Montage der Kugelkopf in dem ersten Formschlusselement 41 aufgenommen ist. Im montierten Zustand ist nur eine Drehung des ersten Formschlusselementes 41 um die Längserstreckung des zweiten Formschlusselementes 42 möglich, alle anderen rotatorischen oder translatorischen Verlagerungen sind gesperrt.

Zum Öffnen des Verschlusssystems und damit zum Lösen des Zugmittels 30 von dem nicht dargestellten Liner 10 wird das erste Formschlusselement 40 von dem zweiten Formschlusselement 42 abgezogen oder durch Anwinkeln getrennt. Werkzeuge sind hierfür nicht notwendig.

Eine Variante des ersten Formschlusselementes 41 ist in der Figur 2a in Seitenansicht und Teilschnittdarstellung gezeigt. Das Formschlusselement 41 weist einen Ausrückmechanismus 45 auf, mit dem es möglich ist, Verriegelungselemente innerhalb des Formschlusselementes 41 durch einen Zug an dem äußeren Kragen außer Eingriff zu bringen, sodass das zu einer Ausnehmung korrespondierende zweite Formschlusselement, das nicht dargestellt ist, ohne oder nur unter leichtem Druck in die Ausnehmung des ersten Formschlusselementes 41 eingeführt und dort verriegelt werden kann. Zum Lösen wird eine entsprechende Entriegelung durch Ziehen an dem Kragen 45 vorgenommen.

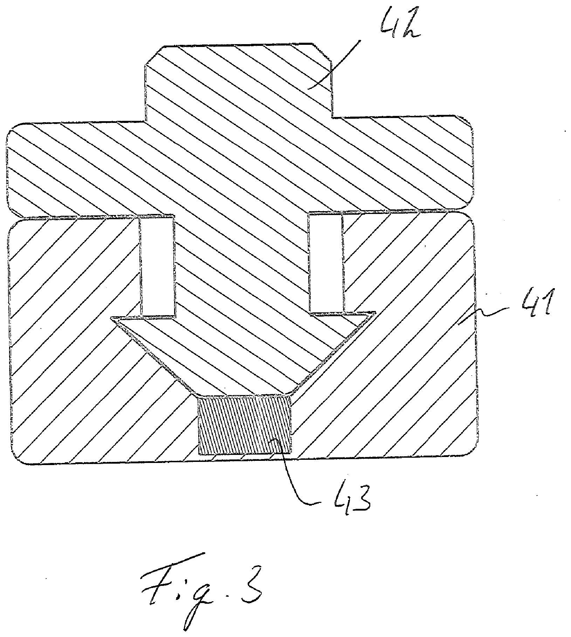

Figur 3 zeigt eine Variante des Verschlusssystems mit den beiden Formschlusselemente 41, 42, im verriegelten Zustand. Statt eines kugelkopfförmigen Formschlusselementes ist eine Verriegelung über einem im kegelstumpfartigen Vorsprung mit Hinterschnitt und einer korrespondierenden Ausnehmung in dem ersten Formschlusselement 41 realisiert. Zusätzlich zu den reinen formschlüssigen Verriegelungen ist eine Magnetsicherung 43 in dem ersten Formschlusselement 41 angeordnet, die das zweite Formschlusselement 42 in die Endposition zieht und darin hält oder zumindest eine zusätzliche Haltekraft aufbringt. Auch hier sind im verriegelten Zustand sämtliche Translationsfreiheitsgrade gesperrt. Bei einer kegelstumpfartigen Ausgestaltung ist ein Rotationsfreiheitsgrad frei, bei einer nicht rotationssymmetrischen Ausgestaltung kann auch dieser gesperrt werden.

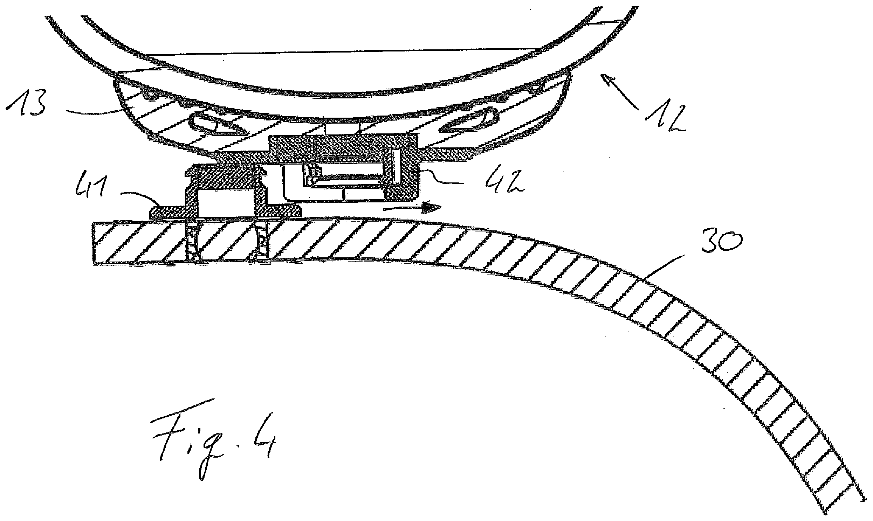

Eine weitere Variante der Erfindung ist in der Figur 4 schematisch dargestellt. Statt einer Verriegelung durch Aufsetzen und Drücken in Einführrichtung des Prothesenliners in dem Prothesenschaft, also in Längserstreckung des Prothesenliners, erfolgt in der Variante gemäß Figur 4 die Verriegelung durch ein Einschieben des ersten Formschlusselementes 41 in das zweite Formschlusselement 42 in einer horizontalen Ebene, also senkrecht zu der Einführrichtung des Prothesenliners 10 in den Prothesenschaft 20. Nach dem Einführen und Verschieben bis an einen Endanschlag kann eine Verriegelung gegen ein unbeabsichtigtes Herausbewegen durch eine federnde Schnappverbindung, eine Drehbewegung oder ein zusätzliches Formschlusselement oder ein Umlegen einer Verriegelung erfolgen.

Nach dem Einführen des ersten Formschlusselementes 41 durch Einschieben in Pfeilrichtung in das zweite Formschlusselement 42 erfolgt eine formschlüssige Verriegelung, sodass eine Fixierung des Zugmittels 30 an dem Prothesenliner 10 erfolgt und ein Trennen senkrecht zu der Einführrichtung des Prothesenliners in den Prothesenschaft nicht möglich ist. Durch die Ausgestaltung des zweiten Formschlusselementes 42 mit einer massiven Seitenwand als Endanschlag wird eine weitergehende Verlagerung nach Erreichen des Endanschlages verhindert.

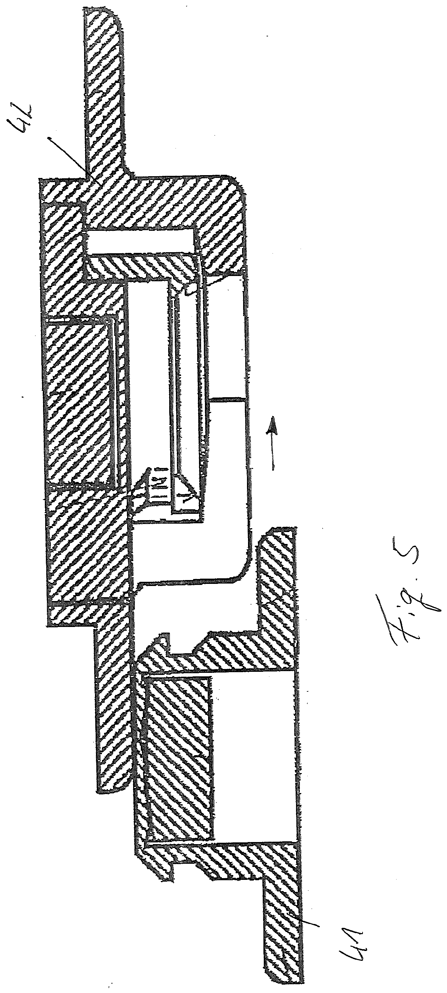

Figur 5 zeigt eine vergrößerte Detaildarstellung des Verschlusssystems 40 mit den beiden Formschlusselementen 41, 42 sowie die Verriegelungsrichtung. Zum Entriegeln kann ein Druck auf das erste Formschlusselement 41 ausgeübt werden, wodurch sich das korrespondierende Formschlusselement aufweitet und eine Freigabe entgegen der Einführrichtung ermöglicht.

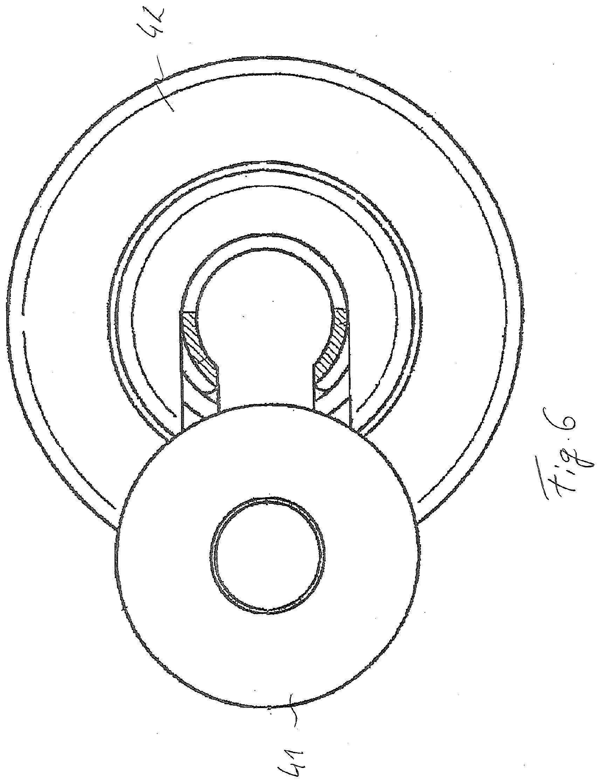

Figur 6 zeigt eine Draufsicht der Formschlusselemente 41, 42 gemäß Figur 5. In dem zweiten Formschlusselement 42 ist ein Einführkanal mit einem Hinterschnitt ausgebildet. Beim Einführen des ersten Formschlusselementes 41 in den Einführkanal werden Federzungen aufgeweitet, die sich nach Erreichen der Entstellung wieder um einen zapfenförmigen Vorsprung herumschließen und eine unbeabsichtigte Entriegelung verhindern, jedoch ein Herausschieben durch Aufbringen einer entgegengesetzt gerichteten Kraft, gegebenenfalls nach Betätigung einer Entsperreinrichtung, ermöglichen.

Alternativ zu den gezeigten Formschlusselementen können diese auch als Klettverschluss ausgebildet sein. Es existieren sehr wirksame Klettverschlussvarianten, mit denen eine hohe Festigkeit der Verbindung erreicht werden kann. Aufgrund der Tatsache, dass nach dem zumindest teilweise Aussteigen aus dem Prothesenschaft 20 eine Abschälbewegung möglich ist, können solche hochfesten Klettverschlüsse auch eingesetzt werden. Ebenfalls können Druckverschlüsse, reine Schiebeverschlüsse und/oder Bajonettverschlüsse, also eine Schiebe-Drehverschlusseinrichtung vorgesehen sein, um werkzeuglos den Prothesenliner 10 mit dem Zugmittel 30 zu verbinden.

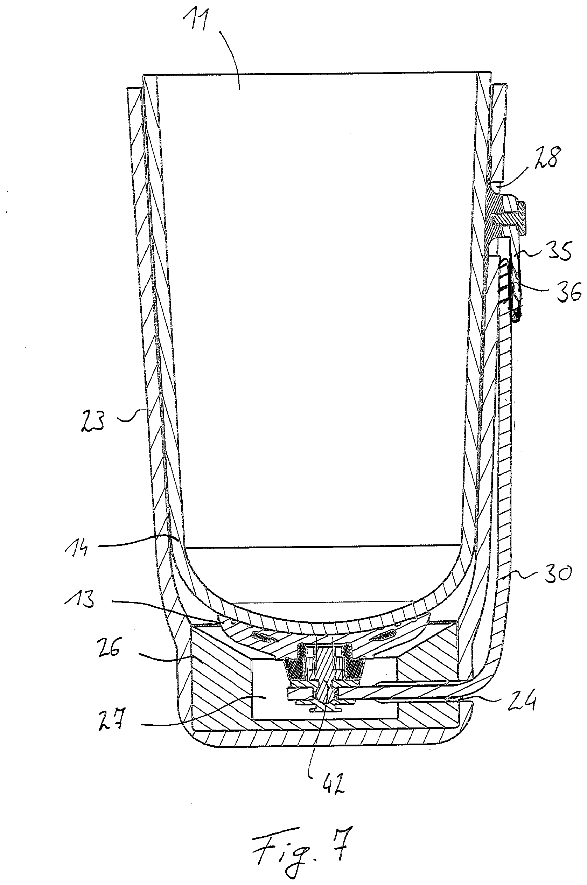

In der Figur 7 ist eine Variante des Prothesenschaftsystems im montierten Zustand in Schnittdarstellung gezeigt. Der Aufbau entspricht im Wesentlichen dem der Figur 1. Es ist zu erkennen, dass der Prothesenliner 10 mit seiner Seitenwand 14 über im Wesentlichen seine gesamte Längserstreckung an der Innenseite der Seitenwand 23 des Prothesenschaftes 20 anliegt. Innerhalb des Fensters 28 in der Seitenwand 23 ist statt eines Umlenkelementes 51 ein zweites Zugmittel 35 angeordnet, das in Gestalt eines Gurtes, einer Lasche oder dergleichen ausgebildet sein kann. Auf der dem Schaft 20 zugewandten Seite des zweiten Zugmittels 35 sind beispielsweise Hakenbereiche eines Klettverschlusses oder gleichwirkende Komponenten angeordnet, während auf der Außenseite, also der dem Prothesenschaft 20 abgewandten Seite an dem ersten Zugmittel 30 die Flauschkomponenten oder korrespondierend wirkende Befestigungseinrichtungen angeordnet sind. Dadurch ist es möglich, die beiden Zugmittel 30, 35 in ihren Endbereichen miteinander zu verbinden, sodass sie aneinander festgelegt werden können. Statt eines Klettverschlusses können auch andere Befestigungseinrichtungen 36 an den Zugmitteln 30, 35 angeordnet sein, beispielsweise eine Schnalle, Löcher für Haken, Bolzen oder andere Vorsprünge, Druckknöpfe oder Klemmeinrichtungen, mit denen das erste Zugmittel 30 an dem zweiten Zugmittel 35 festgelegt werden kann.

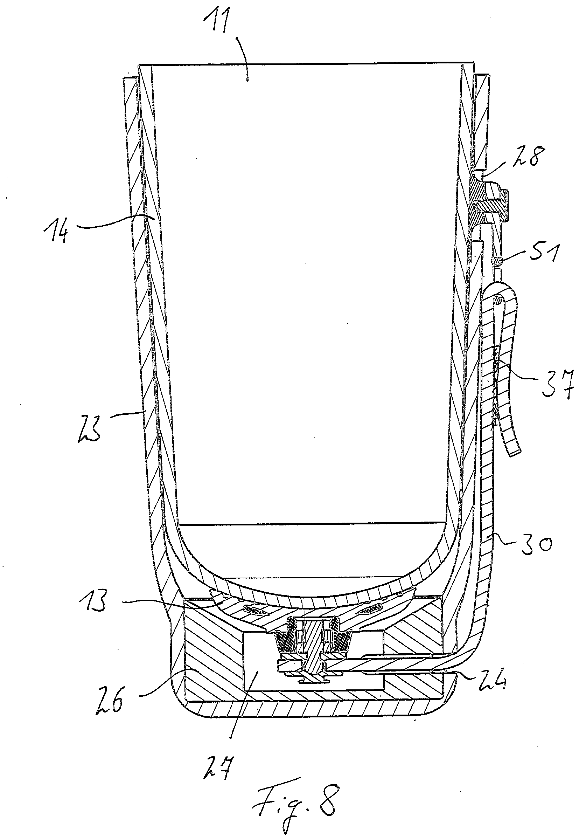

Figur 8 zeigt die Variante gemäß Figur 1 im montierten Zustand. An der Seitenwand 23 des Prothesenschaftes 20 ist ein Umlenkelement 51 befestigt. Durch das Umlenkelement 51 ist das aus dem Prothesenschaft 20 herausgeführte Ende des ersten Zugmittels 30 durchgeführt. Auf der Außenseite des noch nicht durchgeführten Abschnittes des Zugmittels 30 ist eine Komponente eines Klettverschlusses angeordnet. Auf der umgeschlagenen, dann nach innen in Richtung auf den Prothesenschaft zeigenden Seite des umgeschlagenen Endes des ersten Gurtes ist die korrespondierende Komponente des Klettverschlusses angeordnet, sodass nach dem Umschlagen und Umlenken des Zugmittels 30 auf sich selbst über die Einrichtungen 37 mit Hakenbereich und Flauschbereich das Zugmittel 30 in Gestalt eines Gurtes auf sich selbst festgelegt werden kann.

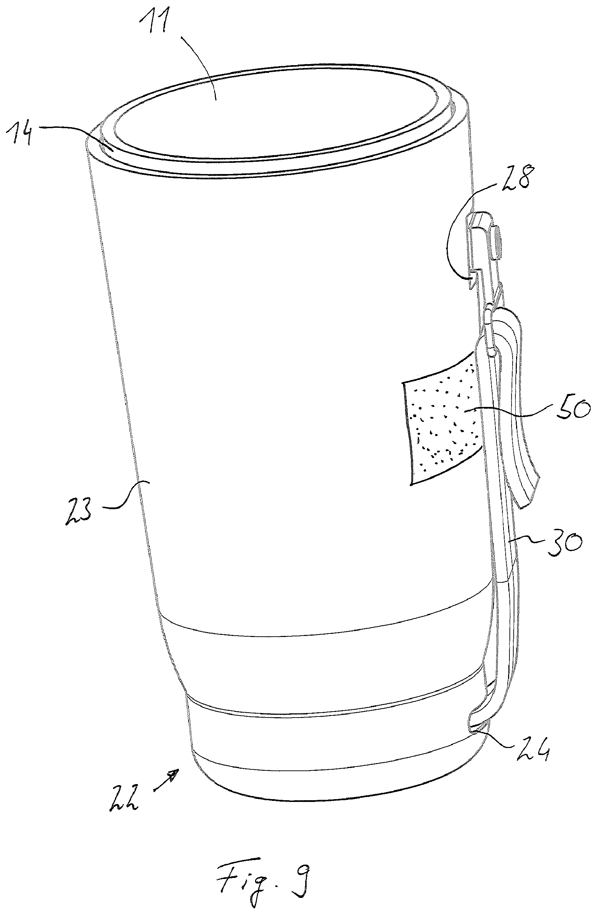

Eine weitere Variante der Erfindung ist in der Figur 9 dargestellt, bei der in dem dargestellten Ausführungsbeispiel das Zugmittel 30 wiederum auf sich selbst fixiert ist, wie in der Figur 8 gezeigt. Zusätzlich ist an der Außenseite der Seitenwand 23 eine Fixiereinrichtung 50 in Gestalt eines Flauschbereiches oder eines Hakenbereiches angeordnet oder ausgebildet, um das Zugmittel 30 entweder nach dem Umlenken durch das Umlenkelement 51 auf der Außenwand festzulegen oder aber um auf das Umlenkelement 51 und damit auch das Fenster 28 verzichten zu können, und das erste Zugmittel 30 unmittelbar an der Seitenwand 23 zu fixieren. Statt einer Fixierung über einen Klettverschluss kann dieses auch über andere Formschlusseinrichtungen erfolgen, beispielsweise durch Löcher in dem Zugmittel 30 und einen oder mehrere Vorsprünge, die in die Löcher einrasten, durch Schnallen oder durch andere formschlüssige oder kraftschlüssige Fixiereinrichtungen 50.

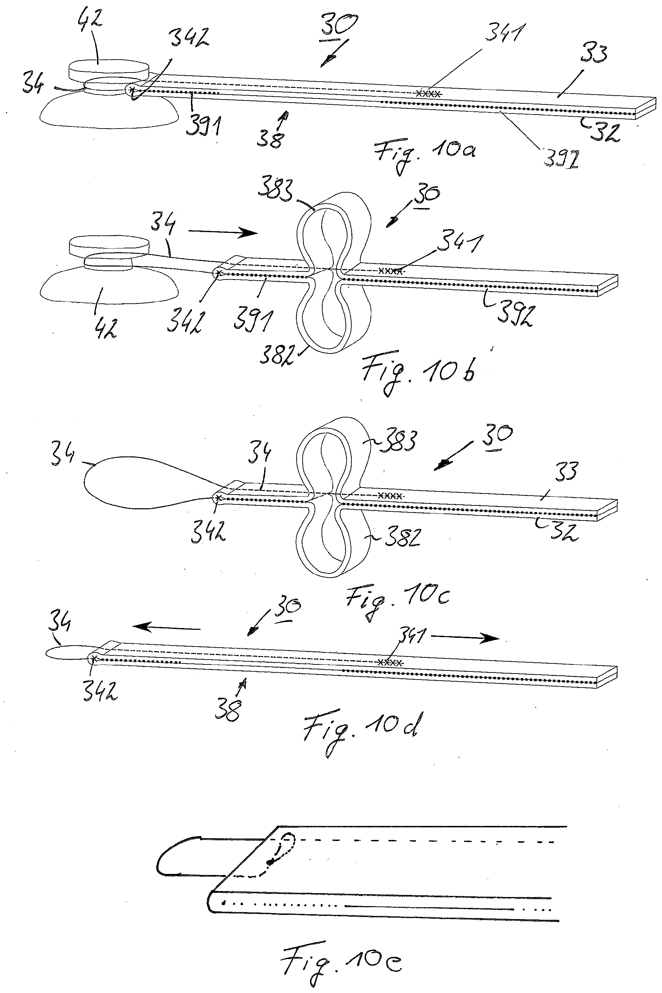

In der Figur 10a ist in einer Detaildarstellung ein Zugmittel 30 dargestellt, das formschlüssig an dem zweiten Formschlusselement 42 eines nicht weiter dargestellten Prothesenliners festgelegt ist. Das Zugmittel 30 ist in dem dargestellten Ausführungsbeispiel als ein Gurt ausgebildet, der auf sich selbst zurückgeschlagen und zweilagig ausgebildet ist. Eine Oberlage 33 und eine Unterlage 32 sind an ihren freien Enden über einen Endbereich mit einer Naht 392 verbunden. Alternativ oder ergänzend können die beiden Lagen 32, 33 verschweißt, verklebt oder auf eine andere Art und Weise miteinander verbunden sein. Im vorderen, umgeschlagenen Bereich des Zugmittels 30 sind die beiden Lagen 32, 33 ebenfalls dauerhaft miteinander verbunden, beispielsweise über eine Naht 391, die als Klebenaht, Schweißnaht oder Nähnaht ausgebildet sein kann. Die Verbindung der beiden Lagen 33, 32 kann in dem Bereich der freien Enden vollflächig ausgebildet sein, in dem Bereich der Umschlagstelle größtenteils vollflächig oder aber auch grundsätzlich nur an dem Umfang befindlich. Zwischen den beiden Nahtbereichen 391, 392 ist ein freier Bereich 38 ausgebildet, in dem die beiden Lagen 32, 33 nicht miteinander verbunden sind, so dass eine Verlagerung der beiden Lagen 32, 33 voneinander weg in diesem Mittelbereich möglich ist.

An dem Zugmittel 30 ist ein Band 34 befestigt, das als Schnur, Kabel, Kordel, Draht, Litze oder Gurt ausgebildet sein kann. Das Band 34 ragt über das umgeschlagene Ende des Zugmittels 30 hinaus und umgibt in dem montierten Zustand das zweite Formschlusselement 42 dergestalt, dass das Zugmittel 30 weder in Längserstreckung des hinterschnittenen Zapfens noch quer dazu von dem zweiten Formschlusselement gelöst werden kann. Das Band 34 ist an zwei Befestigungsstellen 341, 342 an dem Zugmittel 30 fixiert, die erste Befestigungsstelle 341 befindet sich in dem Bereich des Zugmittels 30, der auf der dem zweiten Formschlusselement 42 abgewandten Seite des freien Bereiches 38 befindlichen Abschnittes liegt, während sich die zweite Befestigungsstelle 342 in dem Bereich der Umschlagstelle des Zugmittels 30 befindet. Grundsätzlich ist es auch möglich, dass sich die zweite Befestigungsstelle 342 weiter in Richtung auf den freien Bereich 38 verlagert befindet.

In der Position gemäß Figur 10a ist derjenige Bandabschnitt, der über das umgeschlagene Ende des Zugmittels 30 hinaussteht, so dimensioniert, dass das Zugmittel 30 geradegezogen ist. Beide Lagen 32, 33 liegen auch in dem freien Bereich 38, in dem die beiden Lagen 32, 33 nicht über eine Naht miteinander verbunden sind, aufeinander auf und die durch das überstehende Band 34 gebildete Schlaufe entspricht hinsichtlich ihres Umfanges im Wesentlichen dem umschlungenen zweiten Formschlusselement 42, um so das Zugmittel 30 an dem zweiten Formschlusselement 42 zu fixieren.

Das zweite Formschlusselement 42 ist als pilzförmiger Zapfen mit einem runden Querschnitt ausgebildet, so dass eine Beweglichkeit des Zugelementes 30 um die Längsachse des Zapfens möglich ist. **In** einer alternativen Ausgestaltungsform ist es möglich, den Zapfen unrund auszugestalten oder mit einer Abflachung zu versehen, um keine Rotation in einer Ebene senkrecht zu der Längserstreckungsachse des Zapfens zu ermöglichen, wenn das umgeschlagene Ende des Zugmittels 30 an der Abflachung anliegt.

In der Figur 10b ist das Zugelement 30 in einem Zustand dargestellt, in dem es von dem zweiten Formschlusselement 42 abgenommen werden kann. Dazu wird der vordere, im Bereich der Umschlagstelle gelegene Abschnitt des Zugmittels 30 in Richtung auf den hinteren Abschnitt mit den freien Enden bewegt, so dass das Zugmittel 30 gestaucht wird. Dadurch ergibt sich in dem freien Bereich 38 eine Stauchung, die zu zwei Auswölbungen 382, 383 führt, da der Abstand zwischen der Umschlagstelle des Zugmittels 30 und dem freien Ende verringert wird. Das Band 34 ist an seinem hinteren Ende über die erste Befestigungsstelle 341 an dem hinteren Abschnitt fixiert und in dem vorderen Abschnitt längsbeweglich innerhalb des Zugmittels 30 geführt, so dass bei einer Verlagerung der Umschlagstelle das Band 34 aus dem Zugmittel 30 herausgleiten kann, insbesondere, wenn es noch an dem zweiten Formschlusselement 42 befestigt ist. Dann muss nur das vordere, umgeschlagene Ende des Zugmittels 30 in Pfeilrichtung verlagert werden, wodurch sich die Schlaufe, die durch das Band 34 außerhalb des Zugmittels 30 gebildet wird, vergrößert. **In** dem Zustand gemäß Figur 10b ist es möglich, die durch das Band 34 in dem über die Umschlagstelle hinausstehenden Bereich gebildete Schlaufe von dem zweiten Formschlusselement 42 zu entfernen. Alternativ zu der dargestellten Ausführungsform, bei der das Band 34 nur entlang eines Kanals oder nur mit einem Abschnitt in dem vorderen Bereich des doppellagigen Zugmittels 30 geführt ist, ist es auch möglich, das Band 34 als eine Zuziehschlaufe auszubilden, bei der die Umschlingungsstelle der Zuziehschlaufe in dem Gurt oder Zugmittel 30 liegt, nämlich auf der dem zweiten Formschlusselement 42 abgewandten Seite der Umschlagstelle zwischen der Oberlage 33 und der Unterlage 32. Das Band 34 oder das Kabel ist dabei aus dem Zugmittel 30 an dessen vorderem Ende herausgeführt und bildet die Schlaufe aus, die um das zweite Formschlusselement 42 herumgelegt wird. Hinter dem vorderen Ende des Zugmittels 30 ist das Band 34 oder das Kabel um den beweglich in den vorderen Abschnitt des Zugmittels 30 gelagerten Teil des Bandes 34 oder des Kabels in Form einer Schlaufe herumgelegt, so dass sich eine Zuziehschlaufe ausbildet, bei der durch Ziehen an dem hinteren Ende des Zugmittels 30 die aus dem Zugmittel 30 austretende Schlaufe zugezogen wird. Wird an dem vorderen Ende des Zugmittels 30, also dem Zugmittelabschnitt vor dem freien Bereich 38, von dem zweiten Formschlusselement 42 weg gezogen, vergrößert sich die Schlaufe und kann von dem Formschlusselement 42 abgenommen werden. Eine solche Variante der Erfindung ist in der Fig. 10e gezeigt.

Der verkürzte, zusammengeschobene Zustand, wie er nach dem Zurückziehen des vorderen, umgeschlagenen Endes des Zugmittels 30 erreicht wird, ist in der Figur 10c gezeigt. Die Auswölbungen 382, 383 als freie Abschnitte sind nach außen gerichtet, das Band 34 bildet eine vergleichsweise große Schlaufe, die über die Umschlagstelle hinaussteht. Zum Verringern des Schlaufendurchmessers und damit zum Festlegen des Zugmittels 30 an dem zweiten Formschlusselement 42 über die Schlaufe durch das Band 34 werden die beiden Enden des Zugmittels 30 wieder auseinandergezogen, was in der Figur 10d gezeigt und durch die beiden Pfeile angedeutet ist. Durch das Auseinanderziehen der beiden Zugmittelenden wird auch das Band 34 durch den vorderen Abschnitt des Zugmittels und den freien Bereich 38 hindurch gezogen, so dass sich der Schlaufendurchmesser des Bandes 34 verringert.

In einer vorteilhaften Ausgestaltung ist das Zugmittel 30 mit Rückstelleigenschaften ausgestattet, beispielsweise über eine Materialwahl oder das Integrieren von Rückstellelementen wie Kunststofffedern, Streben, Versteifungen oder dergleichen, so dass eine automatische Rückstellung in den Zustand gemäß Figur 10d erfolgt. Dadurch wird eine permanente Vorspannkraft auf das Band 34 aufgebracht, so dass die Schlaufe und das Zugmittel 30 sicher an dem zweiten Formschlusselement 42 gehalten bleiben. Diese Vorspannkraft kann durch eine erhöhte Knickstabilität des Zugmittelmaterials erreicht werden.

## Patentansprüche

1. Flexibler Prothesenliner (10) mit einer proximalen Einstiegsöffnung (11) für einen Stumpf einer Gliedmaße und einem distalen Endbereich (12) sowie einem flexiblen Zugmittel (30), das in dem distalen Endbereich (12) an dem Prothesenliner (10) angeordnet ist, wobei das Zugmittel (30) über ein werkzeuglos betätigbares Verschlusssystem (40) an dem Prothesenliner (10) lösbar befestigt ist, **dadurch gekennzeichnet, dass** das Verschlusssystem (40) eine Magnetsicherung (43) aufweist.

2. Prothesenliner (10) nach Anspruch 1, **dadurch gekennzeichnet, dass** das Zugmittel (30) formschlüssig und/oder kraftschlüssig an dem Prothesenliner (10) befestigt ist.

3. Prothesenliner (10) nach Anspruch 1, **dadurch gekennzeichnet, dass** an dem Zugmittel (30) ein erstes Formschlusselement (41) angeordnet ist, das mit einem an dem Prothesenliner (10) angeordneten zweiten Formschlusselement (42) in Eingriff tritt.

4. Prothesenliner (10) nach Anspruch 3, **dadurch gekennzeichnet, dass** die Formschlusselemente (41, 42) als Schnappverbindung, Klettverschluss, Druccknopf, Schiebeverschluss und/oder Bajonettverschluss ausgebildet sind.

5. Prothesenliner (10) nach einem der Ansprüche 3 oder 4, **dadurch gekennzeichnet, dass** das Verschlusssystem (40) das Zugmittel (30) entgegen einer Einführrichtung des Prothesenliners (10) aus einem Prothesenschaft (20) blockiert.

6. Prothesenliner (10) nach einem der Ansprüche 3 bis 5, **dadurch gekennzeichnet, dass** das Verschlusssystem (40) in zumindest einer Richtung senkrecht zu der Auszugsrichtung des Prothesenliners (10) aus einem Prothesenschaft (20) das Zugelement (30) blockiert.

7. Prothesenliner (10) nach einem der voranstehenden Ansprüche, **dadurch gekennzeichnet, dass** an dem distalen Endbereich (12) des Prothesenliners (10) ein zusätzliches Sicherungselement zur Festlegung des Prothesenliners (10) an einem Prothesenschaft (20) angeordnet ist.

8. Prothesenschaftsystem mit einem Prothesenliner (10) nach einem der voranstehenden Ansprüche und einem Prothesenschaft (20), mit einer proximalen Einstiegsöffnung (21), einer einen Prothesenliner (10) zumindest teilweise umfänglich umgebenden Seitenwand (23) und einem distalen Abschlussbereich (22), mit Einrichtungen (25) zum Anschließen einer prothetischen Komponente sowie einer Durchtrittsöffnung (24) für das Zugmittel (30) in dem distalen Abschlussbereich (22) des Prothesenschaftes (20).

9. Prothesenschaftsystem nach Anspruch 8, **dadurch gekennzeichnet, dass** an dem distalen Abschlussbereich (22) eine formstabile Abstützeinrichtung (26) mit einer Ausnehmung (27) für das Verschlusssystem (40) angeordnet ist.

10. Prothesenschaftsystem nach Anspruch 8 oder 9, **dadurch gekennzeichnet, dass** an der Außenseite der Seitenwand (23) des Prothesenschaftes (20) eine Fixiereinrichtung (50) für das Zugmittel (30) oder zumindest ein Umlenkelement (51) für das Zugmittel (30), an dem Einrichtungen (37) zum Festlegen auf sich selbst angeordnet sind, angeordnet ist.

11. Prothesenschaftsystem nach einem der Ansprüche 8 bis 10, **dadurch gekennzeichnet, dass** ein Fenster (28) in einem proximalen Bereich der Seitenwand (23) für ein zweites Zugmittel (35) oder zur Festlegung eines Umlenkelementes (51) ausgebildet ist.

12. Prothesenschaftsystem nach Anspruch 11, **dadurch gekennzeichnet, dass** das erste Zugmittel (30) und das zweite Zugmittel (35) Befestigungseinrichtungen (36) zur Festlegung aneinander aufweisen.

## Claims

1. A flexible prosthetic liner (10) with a proximal access opening (11) for a stump of a limb, and a distal end region (12) and also a flexible tensioning means (30) which is arranged on the prosthetic liner (10) in the distal end region (12), wherein the tensioning means (30) is releasably fastened to the prosthetic liner (10) via a closure system (40) which is actuable without a tool, **characterized in that** the closure system (40) has a magnetic securing (43).

2. The prosthetic liner (10) as claimed in claim 1, **characterized in that** the tensioning means (30) is fastened to the prosthetic liner (10) in a form-fitting and/or force-fitting manner.

3. The prosthetic liner (10) as claimed in claim 1, **characterized in that** a first form-fitting element (41) is arranged on the tensioning means (30) and enters into engagement with a second form-fitting element (42) arranged on the prosthetic liner (10).

4. The prosthetic liner (10) as claimed in claim 3, **characterized in that** the form-fitting elements (41, 42) are in the form of a snap-action connection, a hook and loop fastener, a press stud, a sliding closure and/or a quarter-turn fastener.

5. The prosthetic liner (10) as claimed in either of claims 3 and 4, **characterized in that** the closure system (40) blocks the tensioning means (30) counter to an insertion direction of the prosthetic liner (10) from a prosthesis socket (20).

6. The prosthetic liner (10) as claimed in one of claims 3 to 5, **characterized in that** the closure system (40) blocks the tensioning element (30) in at least one direction perpendicular to the pulling-out direction of the prosthetic liner (10) from a prosthesis socket (20).

7. The prosthetic liner (10) as claimed in one of the preceding claims, **characterized in that** an additional securing element for securing the prosthetic liner (10) on a prosthesis socket (20) is arranged at the distal end region (12) of the prosthetic liner (10).

8. A prosthesis socket system with a prosthetic liner (10) as claimed in one of the preceding claims and a prosthesis socket (20), with a proximal access opening (21), a side wall (23) at least partially circumferentially surrounding a prosthetic liner (10), and a distal closing region (22), with devices (25) for connecting a prosthetic component, and a pass-through opening (24) for the tensioning means (30) in the distal closing region (22).

9. The prosthesis socket system as claimed in claim 8, **characterized in that** a dimensionally stable supporting device (26) with a recess (27) for the closure system (40) is arranged at the distal closing region (22).

10. The prosthesis socket system as claimed in claim 8 or 9, **characterized in that** a fixing device (50) for the tensioning means (30) or at least one deflecting element (51) for the tensioning means (30), on which devices (37) for securing same on itself are arranged, is arranged on the outer side of the side wall (23) of the prosthesis socket (20).

11. The prosthesis socket system as claimed in one of claims 8 to 10,
**characterized in that** a window (28) is formed in a proximal region of the side wall (23) for a second tensioning means (35) or for securing a deflecting element (51).

12. The prosthesis socket system as claimed in claim 11, **characterized in that** the first tensioning means (30) and the second tensioning means (35) have fastening devices (36) for securing same to each other.

## Revendications

1. Doublure de prothèse (10) flexible, présentant une ouverture d'entrée proximale (11) pour un moignon d'un membre, et une zone d'extrémité distale (12), ainsi qu'un moyen de traction flexible (30) qui est disposé sur la doublure de prothèse (10) dans la zone d'extrémité distale (12), le moyen de traction (30) étant fixé de manière amovible à la doublure de prothèse (10) via un système de fermeture (40) pouvant être actionné sans outil, **caractérisée en ce que** le système de fermeture (40) comprend un dispositif de retenue magnétique (43).

2. Doublure de prothèse (10) selon la revendication 1,
**caractérisée en ce que** le moyen de traction (30) est fixé à la doublure de prothèse (10) par complémentarité de forme et/ou par adhérence.

3. Doublure de prothèse (10) selon la revendication 1,
**caractérisée en ce qu'**un premier élément de liaison par complémentarité de forme (41) est disposé sur le moyen de traction (30) et s'engage avec un deuxième élément de liaison par complémentarité de forme (42) disposé sur la doublure de prothèse (10).

4. Doublure de prothèse (10) selon la revendication 3,
**caractérisée en ce que** les éléments de liaison par complémentarité de forme (41, 42) sont conçus comme une liaison par encliquetage, une fermeture velcro, un bouton-pression, une fermeture à glissière et/ou une fermeture à baïonnette.

5. Doublure de prothèse (10) selon l'une des revendications 3 ou 4,
**caractérisée en ce que** le système de fermeture (40) bloque le moyen de traction (30) dans le sens opposé à la direction d'insertion de la doublure de prothèse (10) hors d'une emboîture de prothèse (20).

6. Doublure de prothèse (10) selon l'une des revendications 3 à 5,
**caractérisée en ce que** le système de fermeture (40) bloque le moyen de traction (30) dans au moins une direction perpendiculaire à la direction d'extraction de la doublure de prothèse (10) hors d'une emboîture de prothèse (20).

7. Doublure de prothèse (10) selon l'une des revendications précédentes,
**caractérisée en ce qu'**un élément de retenue supplémentaire destiné à immobiliser la doublure de prothèse (10) à une emboîture de prothèse (20) est disposé au niveau de la zone d'extrémité distale (12) de la doublure de prothèse (10).

8. Système d'emboîture de prothèse comprenant une doublure de prothèse (10) selon l'une des revendications précédentes et une emboîture de prothèse (20), présentant une ouverture d'entrée proximale (21), une paroi latérale (23) entourant au moins en partie le pourtour d'une doublure de prothèse (10), et une zone de terminaison distale (22), comprenant des dispositifs (25) pour raccorder un composant prothétique ainsi qu'une ouverture de passage (24) pour le moyen de traction (30) dans la zone de terminaison distale (22) de l'emboîture de prothèse (20).

9. Système d'emboîture de prothèse selon la revendication 8,
**caractérisé en ce qu'**un dispositif de support solide en forme (26) muni d'un évidement (27) pour le système de fermeture (40) est disposé sur la zone de terminaison distale (22).

10. Système d'emboîture de prothèse selon la revendication 8 ou 9,
**caractérisé en ce qu'**un dispositif de fixation (50) pour le moyen de traction (30) ou au moins un élément de renvoi (51) pour le moyen de traction (30), sur lequel sont disposés des organes (37) pour l'immobiliser sur lui-même, est disposé sur la face extérieure de la paroi latérale (23) de l'emboîture de prothèse (20).

11. Système d'emboîture de prothèse selon l'une des revendications 8 à 10,
**caractérisé en ce qu'**une fenêtre (28) est formée dans la zone proximale de la paroi latérale (23) pour faire passer un deuxième moyen de traction (35) ou pour immobiliser un élément de renvoi (51).

12. Système d'emboîture de prothèse selon la revendication 11,
**caractérisé en ce que** le premier moyen de traction (30) et le deuxième moyen de traction (35) comportent des organes de fixation (36) permettant de les immobiliser l'un à l'autre.
